# EUROPÄISCHE PATENTANMELDUNG

(11) **EP 4 505 975 A1**
(43) Veröffentlichungstag der Anmeldung: **12.02.2025**
(21) Anmeldenummer: 23190443.4
(22) Anmeldetag: 09.08.2023
(51) Int. Cl.: A61C 19/04, A61B 5/00

(54) **HANDSTÜCK ZUR BESTIMMUNG PARODONTALER MESSWERTE**

(71) Anmelder: W & H Dentalwerk Bürmoos GmbH, 5111 Bürmoos (AT)
(72) Erfinder: WENDTNER, Wolfgang, 5112 Lamprechtshausen (AT); KOOPMAN, Leon, 5020 Salzburg (AT); LANG, Thomas, 5421 Adnet (AT)
(74) Vertreter: Benda, Ralf

(57) **Zusammenfassung**

Handstück (1, 60) zur Bestimmung parodontaler Messwerte, insbesondere der Tiefe von Zahnfleischtaschen, umfassend: eine Außenhülse (2), eine Bildaufnahmevorrichtung (3), welche durch eine Bildaufnahmeöffnung (4) Bilder eines Zielobjekts aufnimmt, eine Übertragungsvorrichtung (5) zur Übertragung der aufgenommenen Bilder an eine Auswerte- und/ oder Anzeigeeinheit (6) und eine Lagerausnehmung (7) für eine Messsonde (50, 80), wobei die Lagerausnehmung (7) derart ausgebildet ist, dass die Messsonde (50, 80) auf einem Kreisbogen (9) um die Außenhülse (2) verschwenkbar ist. Die Bildaufnahmeöffnung (4) ist in einem Fortsatz (20) an einem ersten Ende (43) der Außenhülse (2) angeordnet, das sich in Richtung des Fortsatzes (20) verjüngende Außenflächen (2A, 16) der Außenhülse (2) umfasst.

Eine Messsonde (50, 80) umfasst einen Lagerabschnitt (51, 81) zur Lagerung an dem Handstück (1, 60), einen Messabschnitt (53, 83) mit einer Biegung zur Bestimmung parodontaler Messwerte und ein Stellelement (52, 85), um die Messsonde (50, 80) auf dem Kreisbogen (9) um die Außenhülse (2) zu verschwenken.

## Beschreibung

Die vorliegende Erfindung betrifft ein Handstück zur Bestimmung parodontaler Messwerte, insbesondere der Tiefe von Zahnfleischtaschen, und eine Messsonde für ein derartiges Handstück zur Bestimmung parodontaler Messwerte, insbesondere der Tiefe von Zahnfleischtaschen.

Ein parodentales Handstück zur Bestimmung parodontaler Messwerte, insbesondere der Tiefe von Zahnfleischtaschen, ist zum Beispiel aus der Anmeldeschrift EP 2 165 674 A1 bekannt. Es umfasst eine Außenhülse, eine Bildaufnahmevorrichtung, welche Bilder eines Zielobjekts, zum Beispiel von Zahnfleischtaschen aufnimmt, eine Übertragungsvorrichtung zur Übertragung der durch die Bildaufnahmevorrichtung aufgenommenen Bilder an eine Auswerte- und/ oder Anzeigeeinheit und eine Messsonde auf.

Der vorliegenden Erfindung liegt die Aufgabe zugrunde die Handhabung eines derartigen parodentalen Handstücks zur Bestimmung parodontaler Messwerte, insbesondere der Tiefe von Zahnfleischtaschen, für den Anwender zu verbessern. Dem Anwender soll zum Beispiel das Einführen der Messsonde in eine Zahnfleischtasche erleichtert werden. Des Weiteren soll beispielsweise auch die Ablesbarkeit einer auf der Messsonde vorgesehenen Messskala verbessert werden.

Diese Aufgabe wird gemäß der vorliegenden Erfindung durch ein Handstück zur Bestimmung parodontaler Messwerte mit den Merkmalen des Anspruchs 1 und 16 und durch eine Messsonde zur Bestimmung parodontaler Messwerte gemäß Anspruch 27 gelöst. Besonders vorteilhafte Ausführungsformen sind in den Unteransprüchen angeführt.

Gemäß einem Ausführungsbeispiel umfasst ein Handstück zur Bestimmung parodontaler Messwerte, insbesondere der Tiefe von Zahnfleischtaschen, eine Außenhülse, eine Bildaufnahmevorrichtung, welche durch eine Bildaufnahmeöffnung des Handstücks Bilder eines Zielobjekts aufnimmt, eine Übertragungsvorrichtung zur Übertragung der durch die Bildaufnahmevorrichtung aufgenommenen Bilder an eine Auswerte- und/ oder Anzeigeeinheit und eine Lagerausnehmung für eine Messsonde, die derart ausgebildet ist, dass die - in der Lagerausnehmung aufgenommene und/ oder gelagerte - Messsonde auf einem Kreisbogen um die Außenhülse verschwenkbar ist. Vorzugsweise umfasst das Handstück zusätzlich eine Messsonde, wobei die in der Lagerausnehmung angeordnete Messsonde auf einem Kreisbogen um die Außenhülse verschwenkbar mit dem Handstück verbunden ist.

Die erfindungsgemäße Ausbildung der Lagerausnehmung und die damit ermöglichte Verschwenkbarkeit der Messsonde relativ zu dem Handstück bewirken in vorteilhafter Weise eine verbesserte Handhabung des Handstücks, da mit Hilfe einer verschwenkbaren Messsonde sowohl linguale als auch faziale Zahnfleischtaschen einfacher erreichbar sind und auch die Sichtbarkeit auf die Messsonde sowohl für den Anwender als auch für die Bildaufnahmevorrichtung verbessert ist.

Gemäß einem anderen Ausführungsbeispiel umfasst ein Handstück zur Bestimmung parodontaler Messwerte, insbesondere der Tiefe von Zahnfleischtaschen, eine Außenhülse, eine Bildaufnahmevorrichtung, welche durch eine Bildaufnahmeöffnung des Handstücks Bilder eines Zielobjekts aufnimmt, eine Übertragungsvorrichtung zur Übertragung der durch die Bildaufnahmevorrichtung aufgenommenen Bilder an eine Auswerte- und/ oder Anzeigeeinheit und eine Lagerausnehmung für eine Messsonde, wobei die Bildaufnahmeöffnung in einem Fortsatz an einem Ende der Außenhülse angeordnet ist und das Ende der Außenhülse durch sich in Richtung des Fortsatzes verjüngende Außenfläche der Außenhülse gebildet ist.

Die sich in Richtung des Fortsatzes verjüngende Außenfläche der Außenhülse und der Fortsatz, der vorzugsweise einen geringeren Außendurchmesser oder Außenumfang als die Außenhülse aufweist, ermöglichen dem Anwender in vorteilhafter Weise eine bessere Handhabbarkeit des Handstücks, insbesondere um damit parodontale Messungen an schwer zugänglichen Bereichen der Mundhöhle oder des Kiefers durchführen zu können. Die sich verjüngenden Außenflächen und die geringeren Außenabmessungen des Fortsatzes mit der Bildaufnahmeöffnung erhöht die Bewegungsfreiheit des Handstücks für den Anwender, zum Beispiel auch eine bessere Drehbarkeit des Handstücks relativ zum Zielobjekt, um somit die Messsonde und die Bildaufnahmevorrichtung optimaler positionieren zu können.

Gemäß einem weiteren Ausführungsbeispiel ist eine Messsonde zur Bestimmung parodontaler Messwerte, insbesondere der Tiefe von Zahnfleischtaschen, vorgesehen, die einen Lagerabschnitt zur Lagerung der Messsonde an dem Handstück und einen daran anschließenden Messabschnitt zur Bestimmung parodontaler Messwerte, insbesondere der Tiefe von Zahnfleischtaschen, umfasst, wobei der Messabschnitt zumindest eine Biegung aufweist, so dass der Messabschnitt in einem Winkel ungleich 0° relativ zu dem Lagerabschnitt bzw. einer Längsachse des Lagerabschnitts angeordnet ist. Die Messsonde ist insbesondere derart ausgebildet, dass sie vom Handstück lösbar ist.

Eine derartige Messsonde erleichtert aufgrund der einen oder mehreren Biegungen des Messabschnitts und des Lagerabschnitts, der eine Verschwenkbarkeit der Messsonde relativ zu einem Handstück, mit dem die Messsonde lösbar verbunden ist, die Positionierung der Messsonde relativ zu dem zu vermessenden Zielobjekt und die Sicht der Bildaufnahmevorrichtung auf die Messsonde.

Das Handstück zur Bestimmung parodontaler Messwerte, insbesondere der Tiefe von Zahnfleischtaschen, ist vorzugsweise als gerades und/oder längliches Handstück ausgebildet, das bzw. dessen Außenhülse sich entlang einer Längsachse erstreckt. Das Handstück und/ oder die Außenhülse weist/weisen vorzugsweise einen Körper auf, der insbesondere als Griffabschnitt ausgebildet ist oder selbigen umfasst.

Ein erstes Ende des länglichen Handstücks oder der Außenhülse weist zumindest eine Bildaufnahmeöffnung sowie eine Messsonde und/ oder eine Lagerausnehmung für eine Messsonde, insbesondere zur lösbaren Aufnahme eine Messsonde, auf. Dieses erste Ende kann entweder einteilig mit der Außenhülse ausgebildet sein oder es kann ein separates Element, zum Beispiel den Fortsatz, umfassen. Bevorzugt weist das erste Ende sich in Richtung des Fortsatzes verjüngende Außenflächen der Außenhülse und/ oder eine verringerte Außenabmessung, insbesondere einen geringeren Außenumfang oder einen geringeren Außendurchmesser als die Außenhülse auf. Die sich verjüngenden Außenflächen liegen bevorzugt am Fortsatz oder an einer Fortsatzöffnung, durch welche sich der Fortsatz erstreckt, an und/ oder begrenzen die Fortsatzöffnung. Durch diese Merkmale wird in vorteilhafter Weise eine bessere Handhabbarkeit des Handstücks erreicht, insbesondere um damit parodontale Messungen an schwer zugänglichen Bereichen der Mundhöhle oder des Kiefers durchführen zu können.

Ein dem ersten Ende gegenüberliegendes Ende kann zum Beispiel als Anschlussende ausgebildet sein. Das Anschlussende weist vorzugsweise eine Verbindungsvorrichtung und/ oder eine Kupplungsvorrichtung und/ oder elektrische Kontakte, zum Beispiel Steckkontakte, auf, an der bzw. an denen eine oder mehrere elektrische Leitungen und/ oder Signalleitungen anschließen. Diese Leitungen und/ oder Signalleitungen verbinden das Handstück vorzugsweise mit einer Auswerte- und/ oder Anzeigeeinheit, die zum Beispiel dazu vorgesehen ist, vom Handstück zur Verfügung gestellte und über die Verbindungsvorrichtung und/ oder Kupplungsvorrichtung und/ oder elektrischen Kontakte und die Leitungen und/ oder Signalleitungen übertragenen Messwerte oder Bilder oder Bilddaten zu verarbeiten und / oder anzuzeigen und/ oder zu speichern. Diese Leitungen und/ oder Signalleitungen können lösbar oder unlösbar mit dem Handstück, insbesondere der Verbindungsvorrichtung und/ oder der Kupplungsvorrichtung und/ oder den elektrischen Kontakten verbunden sein. Alternativ oder zusätzlich können an dem Anschlussende elektrische Kontakte vorgesehen sein, um einen im Handstück angeordneten Energiespeicher, zum Beispiel einen Akkumulator, laden zu können.

Die Außenhülse ist vorzugsweise aus Kunststoff gefertigt, es sind jedoch auch andere Materialien, insbesondere metallische Werkstoffe, denkbar. Die Außenhülse weist vorzugsweise zumindest einen Hohlraum auf, in dem Komponenten des Handstücks aufgenommen sind, zum Beispiel eine Platine, zumindest ein Teil einer Bildaufnahmevorrichtung und/ oder einer Beleuchtungsvorrichtung und/ oder der im Vorstehenden genannten Verbindungsvorrichtung, Kupplungsvorrichtung und/ oder elektrische Kontakte und/ oder elektrische Leiter zur Verbindung der Platine oder darauf angeordneter elektrischer Komponenten mit der Verbindungsvorrichtung, Kupplungsvorrichtung und/ oder den elektrischen Kontakten.

Die Außenhülse kann ein- oder mehrteilig ausgebildet sein, wobei eine mehrteilige Ausbildung in vorteilhafter Weise die Montage der Komponenten im Hohlraum der Außenhülse erleichtert.

Die Außenhülse bildet vorzugsweise auch eine Lagerausnehmung zur Aufnahme und/ oder Lagerung der Messsonde. Damit ist in vorteilhafter Weise kein zusätzliches Bauteil für die Lagerung und/ oder Befestigung der Messsonde an dem parodentalen Handstück notwendig.

Gemäß einem Ausführungsbeispiel ist die Lagerausnehmung an der Außenseite des Handstücks und/ oder der Außenhülse vorgesehen. Vorzugsweise umfasst die Lagerausnehmung einen in der Außenhülse geformten Rücksprung oder eine in der Außenhülse geformte Nut, in dem/ der insbesondere ein Lagerabschnitt oder Verbindungsabschnitt der Messsonde aufnehmbar ist. Vorzugsweise umgibt die an der Außenseite der Außenhülse angeordnete Lagerausnehmung das Handstück kreisbogenförmig. Das Vorsehen der Lagerausnehmung an der Außenseite der Außenhülse ermöglich in vorteilhafter Weise einen sehr einfachen Aufbau des Handstücks ohne zusätzliche Bauteile am Handstück, die für ein Verschwenken der Messsonde notwendig wären.

Gemäß einem anderen Ausführungsbeispiel bildet das Innere der Außenhülse, zum Beispiel der Hohlraum der Außenhülse, zumindest einen Teil der Lagerausnehmung. Die Lagerausnehmung kann dabei Teil eines einzigen von der Außenhülse gebildeten Hohlraums sein. Vorzugsweise ist die Lagerausnehmung jedoch durch einen eigenen Lagerraum gebildet, der zum Beispiel von dem übrigen Hohlraum der Außenhülse durch eine Zwischenwand getrennt ist. Dies verhindert in vorteilhafter Weise das Eindringen von Verschmutzungen über die Lagerausnehmung in den Hohlraum der Außenhülse.

Die Lagerausnehmung oder der Lagerraum ist zur Aufnahme, insbesondere zu einer beweglichen, vorzugsweise drehbaren oder verschwenkbaren, Aufnahme, zumindest eines Teils der Messsonde ausgebildet. Damit kann in vorteilhafter Weise die Handhabbarkeit des Handstücks und die Sichtbarkeit auf das Zielobjekt verbessert werden. Die Lagerausnehmung oder der Lagerraum oder deren Mittelachse erstreckt sich im Wesentlichen parallel zur Längsachse der Außenhülse.

Vorzugsweise weist die Lagerausnehmung oder der Lagerraum eine Öffnung auf, die sich in die Umgebung des Handstücks, insbesondere in einen außerhalb des Handstücks angeordneten Raum oder Sondenraum, öffnet. Durch diese Öffnung der Lagerausnehmung erstreckt sich die Messsonde, insbesondere bildet diese Öffnung die Verbindung des Messabschnitts der Messsonde mit dem Handstück oder dem Lagerraum. Die Öffnung der Lagerausnehmung umgibt die Mittelachse der Lagerausnehmung und/ oder erstreckt sich radial um die Mittelachse. Damit kann in vorteilhafter Weise die Messsonde durch diese Öffnung vom Handstück getrennt werden, zum Beispiel um die Messsonde zu reinigen und/ oder sie gegen eine andere Messsonde oder ein anderes Werkzeug auszutauschen. Natürlich ist es auch denkbar, dass die Lagerausnehmung oder der Lagerraum die Messsonde unlösbar aufnehmen, so dass das Handstück untrennbar mit der Messsonde verbunden ist.

Vorzugsweise weist die Lagerausnehmung oder der Lagerraum eine Innenwand auf, die, wenn die Messsonde mit dem Handstück verbunden ist, die Messsonde, insbesondere einen Lagerabschnitt zur Lagerung der Messsonde an dem Handstück, kontaktiert und/ oder lagert. Vorzugsweise weisen die Innenwand und der Lagerabschnitt, insbesondere dessen Außen- oder Mantelfläche, komplementäre Formen auf. Beispielsweise sind der Lagerabschnitt und die Innenwand beide zylindrisch geformt, wodurch die Messsonde in vorteilhafterweise beim Verschwenken nicht aus der Lagerausnehmung oder dem Lagerraum entnommen werden muss und/ oder jede beliebige Position auf dem Kreisbogen einnehmen kann. Alternativ sind der Lagerabschnitt und die Innenwand beide polygonal geformt, wodurch die Messsonde in vorteilhafterweise beim Verschwenken vorbestimmte Positionen oder Winkel auf dem Kreisbogen um die Außenhülse einnehmen kann.

An dem Handstück ist vorzugsweise eine Haltevorrichtung vorgesehen, die ausgebildet ist, die Messsonde in unterschiedlichen Verschwenkpositionen entlang des Kreisbogens zu halten. Damit wird dem Anwender in vorteilhafter Weise das Einführen der Messsonde in eine Zahnfleischtasche erleichtert, da durch die Haltevorrichtung die Messsonde nicht oder schwerer aus ihrer gewählten Verschwenkposition bewegbar ist.

Die Haltevorrichtung kann Teil eines einzigen von der Außenhülse gebildeten Hohlraums sein, vorzugsweise ist die Haltevorrichtung jedoch durch eine Zwischenwand von dem Hohlraum getrennt, insbesondere von derselben Zwischenwand, welche den Hohlraum von der Lagerausnehmung oder dem Lagerraum trennt, wodurch in vorteilhafter Weise das Eindringen von Verschmutzungen über die Haltevorrichtung in den Hohlraum der Außenhülse verhindert wird. Die Haltevorrichtung und/ oder eine Aufnahme für die Haltevorrichtung ist/ sind vorzugsweise in der Lagerausnehmung oder dem Lagerraum und/ oder anschließend an die Lagerausnehmung oder den Lagerraum angeordnet, wodurch in vorteilhafter Weise eine besonders wirkungsvolle, direkt auf die Messsonde wirkende Fixierung der Messsonde erreichbar ist.

Die Haltevorrichtung ist zum Beispiel als magnetische Haltevorrichtung ausgebildet. Hierzu ist zum Beispiel ein Magnetelement in oder anschließend an die Lagerausnehmung oder den Lagerraum angeordnet. Durch magnetische Wechselwirkung des Magnetelements mit der Messsonde wird die Messsonde in einer beliebigen Verschwenkpositionen entlang des Kreisbogens gehalten. Die Messsonde weist hierzu entweder ebenfalls ein Magnetelement auf, das zum Beispiel in deren Lagerabschnitt angeordnet, zum Beispiel in einen aus Kunststoff hergestellten Lagerabschnitt eingespritzt, oder daran anschließend angeordnet ist, oder der Lagerabschnitt selbst umfasst magnetisches oder magnetisiertes Material. Eine magnetische Haltevorrichtung kann in vorteilhafter Weise einfach im Handstück verbaut werden, ist sehr robust und kann die Messsonde beim Verschwenken in jeder beliebigen Position auf dem Kreisbogen um die Außenhülse halten.

Alternativ oder zusätzlich ist die Haltevorrichtung als mechanische Haltevorrichtung ausgebildet, zum Beispiel als formschlüssige oder kraftschlüssige Haltevorrichtung, als Rast- und/ oder Klemmverbindung, wodurch in vorteilhafter Weise eine zuverlässige Fixierung der Messsonde möglich ist. Eine formschlüssige Haltevorrichtung umfasst zum Beispiel eine oder mehrere Rastelemente, die in der Lagerausnehmung oder dem Lagerraum und der Messsonde, insbesondere an deren Lagerabschnitt, vorgesehen sind und miteinander verrasten. Eine kraftschlüssige Haltevorrichtung umfasst zum Beispiel ein Federelement, dessen Federkraft auf die Messsonde einwirkt und diese gegen einen Abschnitt oder eine Komponente des Handstücks, zum Beispiel die Außenhülse, eine im Folgenden beschriebene Öffnung für das Stellelement in der Außenhülse, insbesondere einen kreisbogenförmigen Schlitz zur Aufnahme des Stellelements der Messsonde oder die Lagerausnehmung, insbesondere deren Innenwand, drückt. Damit ist in vorteilhafter Weise eine Haltevorrichtung geschaffen, welche die Messsonde auch während des Verschwenkens zuverlässig in der Lagerausnehmung oder dem Lagerraum hält.

Gemäß einem besonders bevorzugten Beispiel ist ein Federelement, insbesondere eine Spiralfeder, in einem Federaufnahmeraum, der an die Lagerausnehmung oder den Lagerraum anschließt, angeordnet. Der Federaufnahmeraum ist insbesondere durch Abschnitte der Zwischenwand und/ oder der Außenhülse gebildet. Der Federaufnahmeraum ist insbesondere derart angeordnet, dass er und/ oder das darin aufgenommene Federelement einer Anlagefläche der im Handstück aufgenommenen Messsonde gegenüberliegt/-liegen, wobei die Anlagefläche an einem Ende der Messsonde, insbesondere des Lagerabschnitts, angeordnet ist bzw. dieses Ende bildet. Vorzugsweise drückt das Federelement die Messsonde, insbesondere einen Teil davon, zum Beispiel das am Lagerabschnitt angeordnete Stellelement, gegen das Handstück, insbesondere einen Abschnitt der Außenhülse, um die Messsonde in einer Position auf dem Kreisbogen um die Außenhülse zu halten. Bevorzugt ist ein Druckelement, zum Beispiel eine Kugel oder ein Element mit einer balligen Oberfläche, mit dem Federelement verbunden und dazu vorgesehen, die Federkraft auf die Messsonde, insbesondere den Lagerabschnitt, zu übertragen bzw. zu kontaktieren. An dem Lagerabschnitt, insbesondere der Anlagefläche, kann ein Rücksprung zur Aufnahme zumindest eines Teils des Druckelements vorgesehen sein. Eine Haltevorrichtung mit einem Federelement zum Fixieren der Messsonde benötigt in vorteilhafter Weise kein zusätzlichen Halteelemente an der Messsonde, so dass beliebige Messsonden gehalten werden können.

Die Lagerausnehmung oder der Lagerraum und/ oder die Haltevorrichtung sind bevorzugt derart ausgebildet, dass die in der Lagerausnehmung oder dem Lagerraum aufgenommene Messsonde verschwenkbar ist, während die Haltevorrichtung die Messsonde in der Lagerausnehmung oder dem Lagerraum hält, zum Beispiel die Messsonde formschlüssig fixiert und/ oder eine Haltekraft auf die Messsonde ausübt. Damit ist in vorteilhafter Weise gewährleistet, dass die Messsonde auch während des Verschwenkens in der Lagerausnehmung oder dem Lagerraum fixiert ist bzw. nicht unbeabsichtigt von dem Handstück gelöst wird.

Vorzugsweise ist in der Außenhülse des Handstücks, insbesondere angrenzend an die Lagerausnehmung oder den Lagerraum und/ oder damit in Verbindung stehend, ein Führungsschlitz vorgesehen, der zur Aufnahme und/ oder Führung eines über die Außenhülse ragenden Stellelements der Messsonde vorgesehen ist. Damit kann in vorteilhafter Weise eine Messsonde mit einem über die Außenhülse ragenden Stellelement vom Handstück getrennt bzw. damit verbunden, d.h. in die Lagerausnehmung oder den Lagerraum eingeschoben oder entnommen, werden. Der Führungsschlitz erstreckt sich im Wesentlichen parallel zur Längsachse des Handstücks und/ oder der Außenhülse und/ oder der Lagerausnehmung und/ oder deren Mittelachse. Der Führungsschlitz ist derart ausgebildet, dass er zumindest einen Abschnitt des Stellelements, zum Beispiel einen Schaft oder einen Stift, aufnehmen und/ oder führen kann. Vorzugsweise endet der Führungsschlitz in einer Schlitzöffnung, die sich in die Umgebung des Handstücks, insbesondere in einen außerhalb des Handstücks angeordneten Raum oder Sondenraum, öffnet. Besonders bevorzugt sind die Schlitzöffnung und die Öffnung der Lagerausnehmung oder des Lagerraums nebeneinander angeordnet und/ oder miteinander verbunden und/ oder münden gemeinsam in die Umgebung des Handstücks oder in den Sondenraum.

Gemäß einem Ausführungsbeispiel ist die Messsonde dadurch verschwenkbar, dass ein Anwender die in der Lagerausnehmung oder dem Lagerraum aufgenommene Messsonde oder einen aus dem Handstück ragenden Abschnitt der Messsonde, zum Beispiel einen Messabschnitt zur Bestimmung parodontaler Messwerte oder ein zum Verschwenken vorgesehenes Stellelement, mit einem oder mehreren Fingern oder einem Hilfswerkzeug ergreift und verschwenkt. Vorzugsweise ist die Lagerausnehmung oder der Lagerraum ausgebildet, die Messsonde in mehreren unterschiedlichen Positionen oder Drehwinkeln auf dem Kreisbogen um die Außenhülse zu halten. Insbesondere ist/sind die Lagerausnehmung oder der Lagerraum und/ oder die Messsonde derart ausgebildet, dass die in der Lagerausnehmung oder dem Lagerraum aufgenommene Messsonde verschwenkbar ist, d.h. die Messsonde muss zum Verschwenken nicht aus der Lagerausnehmung oder dem Lagerraum entnommen werden bzw. die Messsonde kann in einer Position, die sie während einer Messung im Lagerraum einnimmt, verschwenkt werden. Alternativ ist es auch denkbar, dass die Messsonde zum Verschwenken aus der Lagerausnehmung oder dem Lagerraum entnommen wird und in einer geänderten Winkelposition wieder in die Lagerausnehmung oder den Lagerraum eingeführt wird. Insbesondere wenn die in der Lagerausnehmung oder dem Lagerraum aufgenommene Messsonde verschwenkbar ist, können zusätzlich an dem Handstück und/ oder der Messsonde eine oder mehrere Vorrichtungen vorgesehen sein, welche die Verschwenkung der in der Lagerausnehmung oder dem Lagerraum aufgenommenen Messsonde für den Anwender erleichtern, so wie dies im Folgenden beschrieben ist.

Vorzugsweise ist in der Außenhülse eine Öffnung vorgesehen, in der das Stellelement der Messsonde verschwenkbar aufgenommen ist, so dass die Messsonde in vorteilhafter Weise auf einem Kreisbogen um die Außenhülse verschwenkbar ist, ohne die Messsonde von dem Handstück zu trennen. Die Öffnung für das Stellelement ist insbesondere eine von der Öffnung der Lagerausnehmung oder des Lagerraums und der Schlitzöffnung unterschiedliche und/ oder davon entfernte, weitere Öffnung. Das Stellelement der Messsonde ragt bevorzugt aus der Öffnung heraus und/ oder erstreckt sich über die Außenhülse hinaus, so dass es in vorteilhafter Weise für den Anwender leicht erreichbar und betätigbar ist. Die Öffnung ist insbesondere dazu vorgesehen, das Stellelement der Messsonde während des Verschwenkens der Messsonde auf einem Kreisbogen um die Außenhülse zu führen und/ oder den Kreisbogen oder Drehwinkel, auf dem die Messsonde verschwenkbar ist, zu begrenzen. Damit wird in vorteilhafter Weise gewährleistet, dass ein Anwender die Messsonde nicht zu weit verschwenken kann, zum Beispiel außerhalb des Sichtfelds der Bildaufnahmevorrichtung.

Die Öffnung ist vorzugsweise als kreisbogenförmiger Schlitz ausgebildet. Die Größe des Winkelbogens der Öffnung oder des kreisbogenförmigen Schlitzes ist vorzugsweise gleich der Größe des maximalen Drehwinkels des Messsonde. Die Öffnung oder der kreisbogenförmige Schlitz, insbesondere deren Winkelbogen, ist vorzugsweise derart bemessen, dass durch das Betätigen des Stellelements der Messabschnitt der Messsonde nur innerhalb des Sichtfelds der Bildaufnahmevorrichtung verschwenkbar ist. Der Winkelbogen beträgt vorzugsweise maximal 130°, vorzugsweise in etwa 90°. Selbstverständlich sind je nach Größe des Sichtfelds, Beschaffenheit des Handstücks, der Messsonde etc. auch andere Winkelbögen ungleich 90° und unterhalb 130° denkbar. Damit wird in vorteilhafter Weise verhindert, dass der Anwender die Messsonde über das Sichtfeld der Bildaufnahmevorrichtung hinausbewegt. Der kreisbogenförmige Schlitz erstreckt sich vorzugsweise radial um die Längsachse des Handstücks bzw. der Außenhülse.

Vorzugsweise ist/ sind die Öffnung für das Stellelement und/ oder die durch die Außenhülse geformten Seitenwände, welche die Öffnung oder den kreisbogenförmigen Schlitz begrenzen, als Widerlager ausgebildet, welches die durch die mechanische Haltevorrichtung, insbesondere die im Vorstehenden beschriebene Haltevorrichtung mit dem Federelement, auf die Messsonde, insbesondere das Stellelement, ausgeübten Kräfte aufnimmt und somit die Messsonde in einer Position auf dem Kreisbogen um die Außenhülse hält. Durch die Ausführung der Öffnung oder des kreisbogenförmigen Schlitzes als Widerlager ist für die zuverlässige Positionierung und Fixierung der Messsonde an dem Handstück kein zusätzliches Lagerelement notwendig.

Vorzugsweise sind der Führungsschlitz und die Öffnung für das Stellelement der Messsonde, insbesondere der kreisbogenförmige Schlitz, miteinander verbunden und/ oder gewinkelt, insbesondere in einem Winkel von etwa 90°, zueinander angeordnet. Vorzugsweise mündet der Führungsschlitz in etwa mittig in den kreisbogenförmigen Schlitz und/ oder der kreisbogenförmige Schlitz erstreckt sich von dem Führungsschlitz zu in etwa oder exakt gleichen Teilen oder mit gleichen Winkelbögen in entgegengesetzte Richtungen. Der maximal in etwa 130° messende Winkelbogen des kreisbogenförmigen Schlitzes erstreckt sich somit vom Führungsschlitz ausgehend maximal jeweils mit in etwa 65° in entgegengesetzte Richtungen. Bei einem Winkelbogen von etwa 90° betragen dem entsprechend die vom Führungsschlitz ausgehenden, sich entgegengesetzt erstreckenden Winkelbogenhälften jeweils in etwa 45°. Vorzugsweise erstreckt sich der Führungsschlitz in der Außenhülse von der Öffnung oder dem kreisbogenförmigen Schlitz bis zu einer Frontseite des Handstücks und/ oder der Schlitzöffnung. Damit kann die Messsonde durch Führung des Stellelements in den beiden Schlitzen in unkomplizierter Weise vom Handstück getrennt bzw. damit verbunden werden.

An dem Handstück ist des Weiteren eine Bildaufnahmevorrichtung vorgesehen, welche durch eine Bildaufnahmeöffnung des Handstücks Bilder eines Zielobjekts aufnimmt. Ein Zielobjekt kann zumindest eines der folgenden Objekte bilden: die Messsonde, insbesondere deren Messabschnitt; zumindest ein Teil eines Zahnfleisches, insbesondere einer Zahnfleischtasche; zumindest ein Teil eines Zahns oder Zahnersatzes; zumindest ein Teil eines Kiefers.

Die Bildaufnahmevorrichtung ist als bekannte Vorrichtung zum Erzeugen eines Bildsignals eines oder mehrerer Zielobjekte ausgebildet. Die Bildaufnahmevorrichtung kann als analoge, vorzugsweise jedoch digitale Vorrichtung, zum Beispiel Kamera, ausgebildet sein. Die Bildaufnahmevorrichtung umfasst insbesondere einen Bildsensor, der insbesondere Bilder im für Menschen sichtbaren Wellenlängenbereich aufnimmt. Der Bildsensor umfasst zum Beispiel einen CCD-Sensor oder einen CMOS-Sensor. Die Bildaufnahmevorrichtung ist ausgebildet Einzelbilder und/ oder Videosequenzen zu erzeugen. Die Bildaufnahmevorrichtung kann des Weiteren eine oder mehrere optische und/ oder elektronische Elemente, wie etwa Linsen oder Filter, aufweisen.

Mit der Bildaufnahmevorrichtung ist operativ und/ oder physisch eine Übertragungsvorrichtung zur Übertragung der durch die Bildaufnahmevorrichtung aufgenommenen Bilder an eine Auswerte- und/ oder Anzeigeeinheit verbunden. Die Übertragungsvorrichtung umfasst vorzugsweise zumindest einen elektrischen Leiter, welcher die von der Bildaufnahmevorrichtung erzeugten Bildsignale an die Auswerte- und/ oder Anzeigeeinheit überträgt. Der zumindest eine elektrische Leiter erstreckt sich zum Beispiel von der Bildaufnahmevorrichtung zu dem Anschlussende des Handstücks und/ oder zu der Verbindungsvorrichtung und/ oder Kupplungsvorrichtung und/ oder elektrischen Kontakten. Von dem Anschlussende verläuft der zumindest eine elektrische Leiter durch ein Kabel zu der Auswerte- und/ oder Anzeigeeinheit, so dass die Bildsignale leitungsgebunden an die Auswerte- und/ oder Anzeigeeinheit übertragen werden. Alternativ ist es auch denkbar, dass die Übertragungsvorrichtung eine Sendeeinrichtung aufweist, welche die Bildsignale drahtlos, zum Beispiel über Funk, an die Auswerte- und/ oder Anzeigeeinheit überträgt. Dazu ist vorzugsweise in dem Handstück eine Sendevorrichtung und an der Auswerte- und/ oder Anzeigeeinheit eine Empfangsvorrichtung vorgesehen.

An dem Handstück ist vorzugsweise eine Beleuchtungsvorrichtung zur Abgabe elektromagnetischer Strahlung zur Beleuchtung des Zielobjekts vorgesehen. Damit wird in vorteilhafter Weise die Qualität der aufgenommenen Bilder erheblich verbessert. Die Beleuchtungsvorrichtung ist vorzugsweise in dem Fortsatz des Handstücks angeordnet. Die Beleuchtungsvorrichtung kann unterschiedliche, bekannte Strahlungsquellen umfassen, insbesondere zumindest einen optischen Halbleiter, eine Leuchtdiode. Die Strahlungsquellen emittiert insbesondere für Menschen sichtbare Wellenlängen, es ist jedoch auch denkbar, dass sie ergänzend Wellenlängen außerhalb des für Menschen sichtbare Wellenlängenspektrums abgibt, um zusätzliche Funktionen, zum Beispiel Kariesdetektion und/ oder Desinfektion, insbesondere in Zahnfleischtaschen, zu ermöglichen. Die Beleuchtungsvorrichtung ist derart am Handstück angeordnet, dass sie zumindest einen Teil, insbesondere einen erheblichen Teil, der von ihr emittierten Strahlung in das Sichtfeld der Bildaufnahmevorrichtung und/ oder in den Sondenraum und/ oder in Richtung der Messsonde, insbesondere des Messabschnitts, abgibt. Damit wird in vorteilhafter Weise eine besonders gute Ausleuchtung des Zielobjekts und/ oder der Messsonde und eine hohe Qualität der Bildsignale erreicht.

An dem Handstück, insbesondere dem Fortsatz, ist jeweils eine Bildaufnahmeöffnung für die Bildaufnahmevorrichtung und eine Beleuchtungsöffnung für die Beleuchtungsvorrichtung vorgesehen. Durch die Bildaufnahmeöffnung gelangt Strahlung von dem Zielobjekt zu der Bildaufnahmevorrichtung bzw. dem Bildsensor zur Erzeugung der Bildsignale. Durch die Beleuchtungsöffnung gelangt Strahlung von der Beleuchtungsvorrichtung in das Sichtfeld der Bildaufnahmevorrichtung und zu dem Zielobjekt. Die Bildaufnahmeöffnung und die Beleuchtungsöffnung sind vorzugsweise getrennte Öffnungen, die durch einen Wandabschnitt des Handstücks und/ oder des Fortsatzes ohne Öffnung verbunden sind. Durch das Vorsehen getrennter Öffnungen werden in vorteilhafter Weise Spiegelungen bei der Bildaufnahme verhindert. Eine, vorzugsweise beide Öffnungen sind durch optische Elemente verschlossen, um in vorteilhafter Weise ein Eindringen von Verschmutzungen in das Handstück zu verhindern. Die optischen Elemente können zum Beispiel in den Öffnungen aufgenommene Glasstopfen sein und/ oder optische Linsen und/ oder optische Filter umfassen. Das optische Element umfasst vorzugsweise Saphirglas. Das optische Element ist zum Beispiel in die Öffnungen eingeklebt oder eingeschmolzen oder eingelötet.

Alternativ ist es auch denkbar, dass die Bildaufnahmeöffnung und die Beleuchtungsöffnung durch eine einzige, gemeinsame Öffnung gebildet werden. Beispielsweise bilden die Beleuchtungsvorrichtung und die Bildaufnahmevorrichtung eine konstruktive Einheit, insbesondere umgibt die Beleuchtungsvorrichtung die Bildaufnahmevorrichtung, vorzugsweise ringförmig, so dass eine einzige, gemeinsame Öffnung für die Beleuchtungsvorrichtung und die Bildaufnahmevorrichtung ausreicht. Eine einzige, gemeinsame Öffnung vereinfacht vorzugsweise die Herstellung des Handstücks.

Zumindest ein Teil der Bildaufnahmevorrichtung und/ oder der Beleuchtungsvorrichtung sind in dem Fortsatz des Handstücks bzw. an einem Ende der Außenhülse und/ oder auf der im Vorstehenden bereits beschriebenen Platine des Handstücks angeordnet, insbesondere auf jenem Abschnitt der Platine, der im Fortsatz des Handstücks aufgenommen ist. Damit kann/ können in vorteilhafterweise die Bildaufnahmevorrichtung und/ oder die Beleuchtungsvorrichtung möglichst nahe an das Zielobjekt herangeführt werden.

Vorzugsweise sind die Bildaufnahmeöffnung und/ oder die Beleuchtungsöffnung in dem Fortsatz des Handstücks bzw. an einem Ende der Außenhülse angeordnet, wobei sich besonders bevorzugt die Messsonde, insbesondere ein Messabschnitt der Messsonde, an oder nahe dem Fortsatz erstreckt. Damit kann/ können in vorteilhafterweise die Bildaufnahmeöffnung und/ oder die Beleuchtungsöffnung möglichst nahe an das Zielobjekt herangeführt werden.

Vorzugsweise ist/ sind die Bildaufnahmeöffnung und/ oder die Beleuchtungsöffnung und/ oder das Sichtfeld der Beleuchtungsvorrichtung und/ oder zumindest Teil der Messsonde radial zu dem Fortsatz einschließlich dessen Mittelachse und/ oder zur Außenhülse einschließlich deren Längsachse und/ oder zum Handstück einschließlich dessen Längsachse angeordnet. Damit ist das Handstück in vorteilhafter Weise für den Anwender einfacher handhabbar, insbesondere im Bereich der Backenzähne.

An dem ersten Ende der Außenhülse des Handstücks und/ oder gegenüber dem Anschlussende des Handstücks ist vorzugsweise eine Frontseite des Handstücks vorgesehen. Die Frontseite ist als Teil der Außenhülse geformt und/ oder schließt an den Griffabschnitt der Außenhülse an. An oder in dieser Frontseite ist zum Beispiel die Öffnung der Lagerausnehmung und/ oder die Schlitzöffnung des Führungsschlitzes vorgesehen. Die Frontseite ist vorzugsweise gewinkelt zum Griffabschnitt der Außenhülse angeordnet. An der Frontseite und/ oder anschließend an die Frontseite ist der Fortsatz des Handstücks angeordnet. Wenn der Fortsatz als separates Teil des Handstücks ausgebildet ist, dann ragt er vorzugsweise aus einer Fortsatzöffnung des Handstücks und/ oder der Außenhülse, die als Teil der Frontseite oder an die Frontseite angrenzend ausgebildet ist.

Der Fortsatz und die Frontseite der Außenhülse begrenzen und/ oder definieren und/ oder bilden vorzugsweise einen außerhalb des Handstücks angeordneten Sondenraum, der von dem Fortsatz überragt ist und in dem zumindest ein Teil der Messsonde, insbesondere ein Teil des Messabschnitts der Messsonde und/ oder des Sichtfelds der Bildaufnahmevorrichtung angeordnet ist. Insbesondere ist der Sondenraum derart geformt und/ oder angeordnet, dass der Sondenraum und/ oder zumindest ein Teil der Messsonde, insbesondere ein Teil des Messabschnitts, innerhalb eines (über die Außenhülse verlängert gedachten) Umfangs der Außenhülse oder des Griffabschnitts angeordnet ist. Vorzugsweise sind das Handstück und/ oder der Fortsatz und/ oder die Frontseite derart ausgebildet, dass zumindest ein Teil der Messsonde, insbesondere der Messabschnitt, in dem Sondenraum auf dem Kreisbogen um die Außenhülse verschwenkbar ist. Die Bildung des Sondenraums, insbesondere durch die sich in Richtung des Fortsatzes verjüngenden Außenflächen der Außenhülse und/ oder unterschiedlichen Außenabmessungen von Außenhülse und Fortsatz, und die damit ermöglichte Anordnung zumindest eines Teils der Messsonde, insbesondere des Messabschnitts, im Sondenraum verbessern die Handhabbarkeit des Handstück in vorteilhafter Weise erheblich, da der Messabschnitt deutlich weniger weit von dem Handstück absteht.

Besonders bevorzugt sind die Bildaufnahmeöffnung und/ oder die Beleuchtungsöffnung derart an dem Fortsatz angeordnet, dass sie in Richtung des Sondenraums zeigen und/ oder daran angrenzen und/ oder sich in den Sondenraum öffnen. Damit sind die Bildaufnahmeöffnung und/ oder die Beleuchtungsöffnung möglichst nahe an dem Messabschnitt der Messsonde, wodurch in vorteilhafter Weise eine genauere Bestimmung der Taschentiefe ermöglicht ist.

Bevorzugt erstreckt sich der Fortsatz in einem Winkel ungleich 0° relativ zu einer Längsachse der Außenhülse und/oder zu der Lagerausnehmung, dem Lagerraum und/ oder zu dem Lagerabschnitt der Messsonde. Vorzugsweise beträgt der Neigungswinkel des Fortsatzes zur Längsachse in etwa 1° - 25°. Die Neigung des Fortsatzes ist insbesondere an die Neigung des Messabschnitts der Messsonde (relativ zur Längsachse des Handstücks und oder der Mittelachse des Lagerabschnitts der Messsonde) angepasst, vorzugsweise derart, dass im Sichtfeld der Bildaufnahmevorrichtung der gesamte Messabschnitt der Messsonde angeordnet ist. Die Neigung des Fortsatzes verbessert somit in vorteilhafter Weise die Bestimmung der Taschentiefe, bei gleichzeitig kürzerem Fortsatz, wodurch wiederum die Handhabbarkeit des Handstücks verbessert wird.

Vorzugsweise ist in dem Fortsatz zumindest ein Teil einer Platine vorgesehen, die sich in dem Fortsatz und der Außenhülse des Handstücks in einem Winkel ungleich 0° relativ zu einer Längsachse der Außenhülse erstreckt und/ oder im Wesentlichen in demselben Winkel wie der Fortsatz relativ zum Handstück angeordnet ist. Auf der Platine ist zum Beispiel ein Teil der Bildaufnahmevorrichtung und/ oder der Beleuchtungsvorrichtung angeordnet. Die Platine und/ oder die Bildaufnahmevorrichtung und/ oder die Beleuchtungsvorrichtung sind somit in entsprechender Weise wie es im vorstehenden Absatz in Bezug auf den Fortsatz beschrieben ist an der Neigung des Messabschnitts der Messsonde orientiert, woraus wiederum dieselben Vorteile resultieren.

Vorzugsweise umfasst der Fortsatz eine metallische Außenhülse. Besonders bevorzugt ist die übrige Außenhülse des Handstücks aus Kunststoff gefertigt. Dies hat den Vorteil, dass die optischen Elemente, welche wie im Vorstehenden beschrieben, die Bildaufnahmeöffnung und die Beleuchtungsöffnung verschließen, dauerhaft dicht in den metallischen Fortsatz eingefügt werden können, zum Beispiel durch Löten oder Einglasen, wohingegen der Griffabschnitt aus Kunststoff für den Anwender angenehmer zu halten und leichter ist. Besonders bevorzugt ist der metallische Fortsatz ein vom Griffabschnitt der Außenhülse separates Teil, das in die Fortsatzöffnung der Außenhülse oder der Frontseite oder angrenzend an diese eingesetzt ist, so wie dies im Vorstehenden bereits beschrieben ist. Damit wird in vorteilhafter Weise auch die Montage der Platine im Inneren des Fortsatzes und der Außenhülse erleichtert. Der Fortsatz ist bevorzugt als Hohlkörper ausgebildet, in und/ oder an den unterschiedlichen Komponenten des Handstücks angeordnet sind, zum Beispiel zumindest ein Abschnitt der Platine, Teile der Beleuchtungs- und/ oder Bildaufnahmevorrichtung, der Übertragungsvorrichtung, die Bildaufnahme- und/ oder Beleuchtungsöffnung, ein Vergussmaterial und andere Elemente. Der Fortsatz ist an seiner Außenseite zum Beispiel quaderförmig, rotationssymmetrisch oder zylindrisch geformt.

Vorzugsweise ist das Innere des Fortsatzes und/ oder zumindest ein Teil der Außenhülse mit einem Vergussmaterial gefüllt. Das Vergussmaterial umfasst zum Beispiel Epoxid-Vergussmaterial, Silikon-Vergussmaterial oder andere bekannte Vergussmaterialien. Das Ausfüllen des Fortsatzes und/ oder zumindest eines Teils der Außenhülse bewirkt auch ein Umhüllen und/ oder Vergießen der darin enthaltenen Komponenten, insbesondere der im Vorstehenden genannten elektrischen Komponenten wie Platine, Bildaufnahmevorrichtung, Beleuchtungsvorrichtung, Übertragungsvorrichtung und andere Bauteile, und damit einen Schutz dieser vergossenen Komponenten vor externen Einflüssen, Verunreinigungen, Reinigungsmedien etc. Das Vergussmaterial verbindet insbesondere auch den Fortsatz mit der Außenhülse und dichtet diese Schnittstelle sowie gegebenenfalls weitere Schnittstellen, zum Beispiel der Außenhülse, zur Umgebung hin ab. Durch das Vorsehen der im Vorstehenden beschriebenen Zwischenwand, welche die Lagerausnehmung oder den Lagerraum von dem übrigen Innenraum der Außenhülse trennt, ist beim Vergießen des Innenraums sichergestellt, dass kein Vergussmaterial in die Lagerausnehmung oder den Lagerraum gelangt. Die Lagerausnehmung oder der Lagerraum sind somit frei von Vergussmasse, um die Verschwenkbarkeit der Messsonde in der Lagerausnehmung auf einem Kreisbogen um die Außenhülse sicherzustellen.

Die Messsonde weist vorzugsweise einen Lagerabschnitt auf, der verschwenkbar in der Lagerausnehmung des Handstücks anordenbar ist. Der Lagerabschnitt ist insbesondere länglich geformt. Der Lagerabschnitt ist insbesondere rotationssymmetrisch geformt, zum Beispiel zylindrisch, wodurch die Messsonde in vorteilhafter Weise jede beliebige Position auf dem, insbesondere durch den kreisbogenförmigen Schlitz definierten, Kreisbogen einnehmen kann. Alternativ ist der Lagerabschnitt polygonal geformt, wodurch die Messsonde in vorteilhafterweise beim Verschwenken vorbestimmte Positionen oder Winkel auf dem Kreisbogen um die Außenhülse einnehmen kann. An den Lagerabschnitt, insbesondere an eine erste Grundfläche des zylindrischen Lagerabschnitts, schließt ein der Messabschnitt der Messsonde an.

Vorzugsweise ist an einem Ende des Lagerabschnitts, insbesondere an jenem Ende, das dem Ende mit dem daran anschließenden Messabschnitt gegenüber angeordnet ist, eine Anlagefläche vorgesehen. Vorzugsweise ist die Anlagefläche durch eine zweite Grundfläche, welche der ersten Grundfläche des zylindrischen Lagerabschnitts gegenüberliegt, gebildet. Wie im Vorstehenden bereits beschrieben, ist die Anlagefläche dazu vorgesehen, dass die Haltevorrichtung des Handstück die Messsonde kontaktiert, um die Messsonde in dem Handstück, insbesondere in unterschiedlichen Verschwenkpositionen relativ zu dem Handstück, zu halten. Durch die Verwendung der Grundfläche als Anlagefläche für die Haltevorrichtung ist es in vorteilhafter Weise nicht notwendig, separate Komponenten an der Messsonde vorzusehen, an denen die Haltevorrichtung eingreifen kann.

Die Messsonde, insbesondere der Lagerabschnitt, weisen bevorzugt ein Stellelement auf, das durch die Öffnung für das Stellelement der Außenhülse ragt und relativ zur Außenhülse verschwenkbar ist, um die Messsonde auf dem Kreisbogen um die Außenhülse zu verschwenken, so wie dies im Vorstehenden bereits beschrieben ist. Das Vorsehen eine Stellelements an der Messsonde erleichtert dem Anwender das Verschwenken der Messsonde auf dem Kreisbogen um die Außenhülse und ist hygienisch vorteilhaft, da, wenn das Stellelement nicht vorhanden wäre, der Anwender genötigt wäre zum Verschwenken den Messabschnitt der Messsonde zu berühren. Vorzugsweise sind das Stellelement und der Führungsschlitz des Handstücks derart ausgebildet, dass das Stellelement auch in den Führungsschlitz und insbesondere darüber hinausragt, so dass auch das Einführen der Messsonde in und das Lösen der Messsonde aus dem Handstück mithilfe des Stellelements und durch das Führen in dem Führungsschlitz vereinfacht wird, so wie dies im Vorstehenden bereits beschrieben ist.

Das Stellelement umfasst zum Beispiel einen Schaft oder Stift, der insbesondere derart bemessen ist, dass er in der Öffnung für das Stellelement, dem kreisbogenförmigen Schlitz und vorzugsweise dem Führungsschlitz aufnehmbar und bewegbar ist. Das Stellelement ist an der Messsonde, insbesondere dem Lagerabschnitt, befestigt, insbesondere einteilig damit ausgebildet. Das Stellelement ist vorzugsweise an einem Ende des Lagerabschnitts, insbesondere an dem die Anlagefläche aufweisenden Ende, vorgesehen. Das Stellelement erstreckt sich in etwa rechtwinkelig von der Messsonde, insbesondere dem Lagerabschnitt. An dem freien Ende des Stellelements, das insbesondere über die Außenhülse und die Öffnung für das Stellelement und den Führungsschlitz ragt, ist vorzugsweise eine Verdickung, zum Beispiel eine knopf- oder höckerartige Verdickung, vorgesehen, deren Außendurchmesser größer ist als der Außendurchmesser des Schafts oder Stifts des Stellelements, wodurch die Bedienbarkeit des Stellelements für den Anwender noch weiter vereinfacht ist.

Der an den Lagerabschnitt anschließende Messabschnitt der Messsonde zur Bestimmung parodontaler Messwerte, insbesondere der Tiefe von Zahnfleischtaschen, ist stiftförmig und/ oder zylindrisch ausgebildet. Der Messabschnitt erstreckt sich vom Lagerabschnitt wegweisend und endet insbesondere in einer Spitze, die vorzugsweise konisch und am Ende abgerundet ist, um das Eindringen in Zahnfleischtaschen zu erleichtern. Am freien Vorderende oder der Spitze können Skalierung oder Markierungen vorgesehen sein, um die Eindringtiefe in eine Zahnfleischtasche messen zu können.

Vorzugsweise umfasst der Messabschnitt zumindest eine Biegung, so dass der Messabschnitt in einem Winkel ungleich 0° relativ zu dem Lagerabschnitt angeordnet ist. Vorzugsweise erstrecken sich die Messsonde, insbesondere der Messabschnitt der Messsonde, und/ oder das Sichtfeld der Bildaufnahmevorrichtung in einem Winkel ungleich 0° von der Längsachse der Außenhülse. Durch diese Maßnahmen wird die Positionierung der Messsonde relativ zu dem zu vermessenden Zielobjekt erleichtert und die Sicht der Bildaufnahmevorrichtung auf die Messsonde verbessert.

Die Messsonde ist vorzugsweise aus Kunststoff und/ oder Metall gefertigt, wobei insbesondere der Messabschnitt aus Kunststoff hergestellt ist, um eine schonende Messung, insbesondere der Zahnfleischtiefe, zu gewährleisten. Der Lagerabschnitt ist entweder aus Kunststoff und/ oder Metall gefertigt, wobei ein metallischer Lagerabschnitt in vorteilhafter Weise einen geringeren Verschleiß durch das Verschwenken aufweist.

Die Erfindung wird nachfolgend anhand bevorzugter Ausführungsbeispiele und Bezug nehmend auf die beigefügten Zeichnungen erläutert. Es zeigt die
Figur 1 eine Außenansicht eines Handstück zur Bestimmung parodontaler Messwerte mit einer in der Lageraufnahme des Handstücks eingeführten und verschwenkten Messsonde;
Figur 2 das Handstück der Figur 1 ohne Messsonde in Vorderansicht;
Figur 3 eine Schnittdarstellung des Handstücks und der Messsonde der Figur 1;
Figur 4 eine Außenansicht eines Handstück zur Bestimmung parodontaler Messwerte in Vorderansicht mit eingeführter und in eine erste Richtung verschwenkter Messsonde mit zwei unterschiedlichen Verschwenkwinkeln;
Figur 5 eine Außenansicht eines Handstück zur Bestimmung parodontaler Messwerte in Vorderansicht mit eingeführter und in eine zweite Richtung verschwenkter Messsonde mit zwei unterschiedlichen Verschwenkwinkeln;
Figur 6 eine Messsonde zur Bestimmung parodontaler Messwerte, insbesondere zur Messung der Tiefe von Zahnfleischtaschen;
Figur 7 eine Außenansicht eines Handstück zur Bestimmung parodontaler Messwerte gemäß einem alternativen Ausführungsbeispiel;
Figur 8 eine Messsonde zur Bestimmung parodontaler Messwerte, insbesondere zur Messung der Tiefe von Zahnfleischtaschen, gemäß einem alternativen Ausführungsbeispiel.

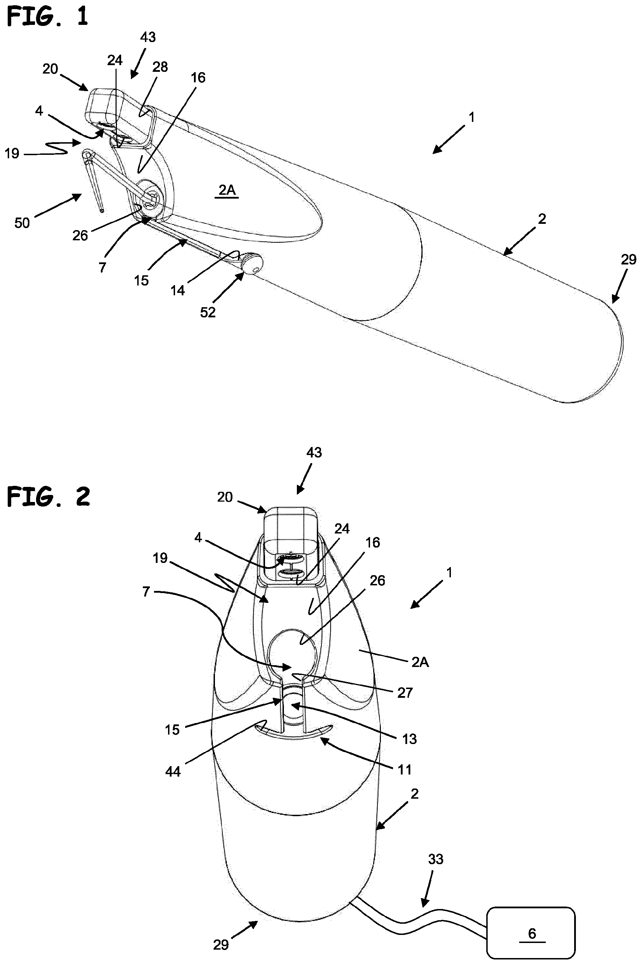

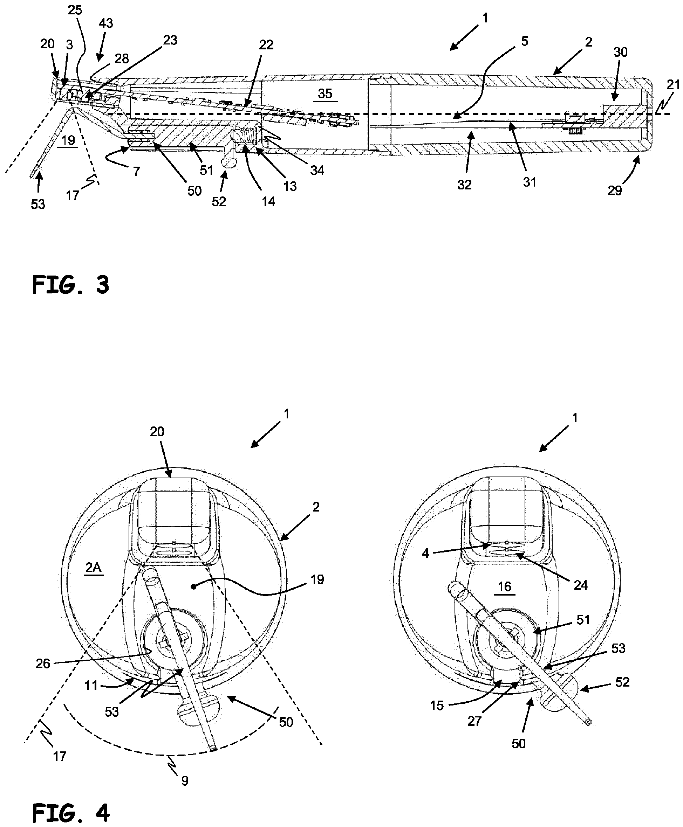

Die in den Figuren 1 - 5, 7 abgebildeten Handstücke 1, 60 zur Bestimmung parodontaler Messwerte, insbesondere der Tiefe von Zahnfleischtaschen, umfassen eine Außenhülse 2, eine Bildaufnahmevorrichtung 3, siehe insbesondere Figur 3, welche durch eine Bildaufnahmeöffnung 4 des Handstücks 1, 60 Bilder eines Zielobjekts aufnimmt, eine Übertragungsvorrichtung 5 zur Übertragung der durch die Bildaufnahmevorrichtung 3 aufgenommenen Bilder an eine Auswerte- und/ oder Anzeigeeinheit 6, siehe Figur 2, und eine Lagerausnehmung 7 für eine Messsonde 50, 80 zur Bestimmung parodontaler Messwerte, insbesondere der Tiefe von Zahnfleischtaschen. Das Handstück 1, 60 ist als gerades, längliches Handstück 1, 60 ausgebildet, das bzw. dessen Außenhülse 2 sich entlang einer Längsachse 21, siehe Figur 3, erstreckt.

Die Außenhülse 2 ist vorzugsweise mehrteilig aufgebaut. Zumindest ein Abschnitt der Außenhülse 2 bildet eine Griffabschnitt zum manuellen Halten des Handstücks 1, 60 durch einen Anwender. Der Griffabschnitt kann zumindest teilweise eine von der übrigen Außenhülse 2 abweichende Außenform aufweisen, um das Halten zu erleichtern. Während die Außenhülse 2 zylindrisch geformt ist, kann zumindest ein Teil des Griffabschnitts Bereiche 2A aufweisen, die sich insbesondere in Richtung des Vorderendes oder einer Frontseite 16 des Handstücks 1, vorzugsweise keilförmig, annähern und auf die Frontseite 16 treffen. Diese Bereiche 2A sind vorzugsweise abgeflacht oder eben ausgebildet. Die Frontseite 16 der Handstücke 1, 60 ist gewinkelt zur Außenhülse 2 angeordnet.

Ein erstes Ende 43 der Handstücke 1 der Figuren 1 - 5 umfasst die Frontseite 16, eine Öffnung 26 der Lagerausnehmung 7 und eine Schlitzöffnung 27 eines Führungsschlitzes 15. An dem ersten Ende 43 des Handstücks 1, 60 anschließend an die Frontseite 16 ist des Weiteren ein Fortsatz 20 des Handstücks 1, 60 angeordnet. Der Fortsatz 20 ist als von der Außenhülse 2 bzw. dem abgeflachten Bereich 2A des Griffabschnitts separates Teil des Handstücks 1, 60 ausgebildet und ragt aus einer Fortsatzöffnung 28 des Handstücks 1, 60 und/ oder der Außenhülse 2. Bei den Handstücken 1 der Figuren 1 - 5 erstreckt sich die Frontseite 16, insbesondere gebogen, zwischen der Fortsatzöffnung 28 und der Öffnung 26 der Lagerausnehmung 7 bzw. der Schlitzöffnung 27.

Ein dem ersten Ende 43 gegenüberliegendes, zweites Ende des Handstücks 1, 60 ist als Anschlussende 29 ausgebildet. Das Anschlussende 29 weist vorzugsweise eine Verbindungs- und/ oder Kupplungsvorrichtung 30 auf, an die elektrische Leitungen 31 und eine Signalleitung 32 anschließen, die sich von der Verbindungs- und/ oder Kupplungsvorrichtung 30 durch das Innere des Handstücks 1, 60 bzw. der Außenhülse 2 bis zur Bildaufnahmevorrichtung 3 bzw. einer Beleuchtungsvorrichtung 23 erstrecken. Die elektrischen Leitungen 31 sind ausgebildet, die Bildaufnahmevorrichtung 3 und die Beleuchtungsvorrichtung 23 zum Betrieb mit elektrischer Energie zu versorgen. Die Signalleitung 32, die insbesondere Teil der Übertragungsvorrichtung 5 ist, ist ausgebildet, von der Bildaufnahmevorrichtung 3 erzeugte Bilddaten oder Bilder zu übertragen. Die Verbindungs- und/ oder Kupplungsvorrichtung 30 ist dazu vorgesehen, das Handstück 1, 60 insbesondere die elektrische Leitungen 31 und die Signalleitung 32, mit einer Auswerte- und/ oder Anzeigeeinheit 6 zu verbinden. Dazu ist ein Kabel 33 vorgesehen, durch welches die elektrischen Leitungen 31 und die Signalleitung 32 bis zur Auswerte- und/ oder Anzeigeeinheit 6 geführt werden. Die Verbindungs- und/ oder Kupplungsvorrichtung 30 ist lösbar ausgebildet, so dass das Kabel 33 von dem Handstück 1, 60 getrennt werden kann. Die Auswerte- und/ oder Anzeigeeinheit 6 ist dazu vorgesehen, die übertragenen Bilder oder Bilddaten zu verarbeiten und / oder auf einem Bildschirm anzuzeigen und/ oder zu speichern. Die Auswerte- und/ oder Anzeigeeinheit 6 umfasst des Weiteren eine Energiequelle oder ist mit einer Energiequelle verbunden, um elektrische Energie an über die elektrische Leitung 31 an das Handstück 1, 60 zu übertragen.

Der Fortsatz 20 und die Frontseite 16 begrenzen und/ oder definieren und/ oder bilden einen außerhalb des Handstücks 1, 60 angeordneten Sondenraum 19, der von dem Fortsatz 20 überragt ist. In dem Sondenraum 19 ist zumindest ein Teil der Messsonde 50, 80 insbesondere ein Teil eines Messabschnitts 53, 83 der Messsonde 50, 80 und/ oder des Sichtfelds 17 der Bildaufnahmevorrichtung 3 angeordnet.

Die in der Außenhülse 2 angeordnete Lagerausnehmung 7 und der durch die Lagerausnehmung 7 gebildete Lagerraum sind zur beweglichen, verschwenkbaren Aufnahme und Lagerung der Messsonde 50, 80 vorgesehen. Die Lagerausnehmung 7 ist durch eine Zwischenwand 34 vom Innenraum 35 der Außenhülse 2 getrennt, wobei der Innenraum 35 vorzugsweise von der Umgebung verschlossen und/ oder abgedichtet ist.

Im Folgenden werden Merkmale der Handstücke 1 der Figuren 1 - 5 beschrieben.

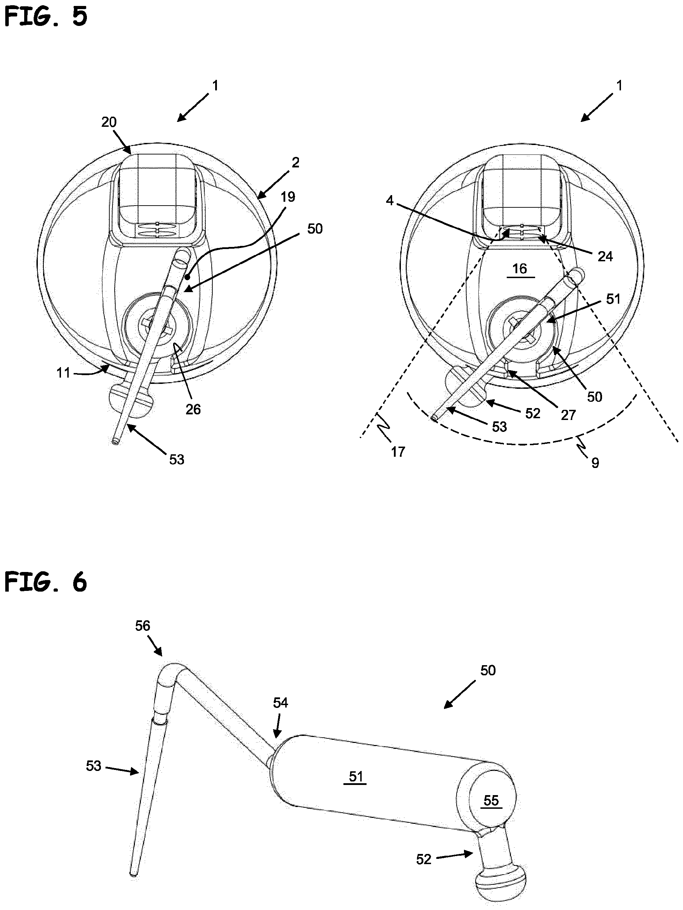

An der Frontseite 16 mündet die Lagerausnehmung 7 in die Öffnung 26, die sich in den Sondenraum 19 öffnet bzw. die Lagerausnehmung 7 mit dem Sondenraum 19 verbindet. Durch die Öffnung 26 erstreckt sich die Messsonde 50 bzw. kann die Messsonde 50, insbesondere deren Lagerabschnitt 51, in das Handstück 1 eingeführt und davon getrennt werden. Sowohl der Lagerabschnitt 51 als auch die Innenwand der Lagerausnehmung 7 sind zylindrisch geformt, so dass die Messsonde 50 gleitend in beliebige Drehpositionen auf einem Kreisbogen 9 um die Außenhülse 2 verschwenkbar ist, siehe Figuren 4 und 5 mit jeweils einer schematischen Darstellung eines Kreisbogens 9. Der Kreisbogen 9, entlang dem die Messsonde verschwenkbar ist, ist im Sondenraum 19 und im Sichtfeld 17 der Bildaufnahmevorrichtung 3 angeordnet.

Um das Verschwenken der Messsonde 50 auf dem Kreisbogen 9 zu erleichtern, ist in der Außenhülse 2 eine Öffnung oder ein Schlitz 11, insbesondere ein kreisbogenförmiger Schlitz, für ein Stellelement 52 der Messsonde 50 vorgesehen. Die Öffnung 11 nimmt das Stellelement 52 auf und führt dieses in einem der Außenhülse 2 folgenden Kreisbogen. Das stiftförmige Stellelement 52 erstreckt sich aus der Lagerausnehmung 7 und ragt durch die Öffnung 11 in die Umgebung, wobei das freie Ende des Stellelement 52 eine kugelige Verdickung aufweist, so dass das Stellelement 52 für den Anwender besser bedienbar ist.

Das Handstück 1 weist des Weiteren eine Haltevorrichtung 13 auf, um die Messsonde 50 in der Lagerausnehmung 7 zu halten, insbesondere in unterschiedlichen Verschwenkpositionen entlang des Kreisbogens 9 zu halten, siehe Figur 3. Die Haltevorrichtung 13 ist in einer an die Lagerausnehmung 7 anschließenden Aufnahme 14 angeordnet. Die Aufnahme 14 ist durch die Zwischenwand 34 von dem Innenraum 35 der Außenhülse 2 getrennt und erstreckt sich vorzugsweise axial zu einer Mittelachse der Lagerausnehmung 7. Gemäß Figur 3 umfasst die Haltevorrichtung 13 ein Federelement und ein Druckelement, zum Beispiel eine Kugel, das an dem Federelement gelagert ist und die Federkraft des Federelements auf die Messsonde 50, insbesondere den Lagerabschnitt 51, überträgt. Die Haltevorrichtung 13 ist derart ausgebildet, dass die in der Lagerausnehmung 7 aufgenommene Messsonde 50 verschwenkbar ist, während die Haltevorrichtung 13 die Messsonde 50 in der Lagerausnehmung 7 hält, insbesondere eine Federkraft auf die Messsonde 50 ausübt. Es ist somit kein Lösen der Messsonde 50 vom Handstück und/ oder aus einer Messposition, welche die Messsonde während eines Messvorgangs einnimmt, notwendig, um die Messsonde 50 verschwenken zu können.

Die Öffnung 11 für das Stellelement 52, insbesondere der die Öffnung 11 begrenzende oder umgebende Rand, der durch die Außenhülse 2 und/ oder Frontseite 16 gebildet ist, ist als Widerlager 44 ausgebildet ist, welches die durch die Haltevorrichtung 13 auf die Messsonde 50 ausgeübten Kräfte aufnimmt. Die Messsonde 50, insbesondere das Stellelement 52, ist somit durch die Haltevorrichtung 13 gegen den Rand der Öffnung 11, d.h. Widerlager 44 gedrückt, wodurch ein Lösen der Messsonde 50 vom Handstück 1 verhindert wird. Die Öffnung 11 für das Stellelement 52 der Messsonde 50 ist derart bemessen, dass durch das Betätigen des Stellelements 52 der Messabschnitt 53 der Messsonde 50 nur innerhalb des Sichtfelds 17 der Bildaufnahmevorrichtung 3 verschwenkbar ist.

Der Führungsschlitz 15 erstreckt sich in der Außenhülse 2 von dem Schlitz 11 für das Stellelement 52 der Messsonde 50 bis zu einer Frontseite 16 des Handstücks. Der Führungsschlitz 15 endet in der Schlitzöffnung 27, die in den Sondenraum 19 mündet. Durch diesen Führungsschlitz 15 ist das Stellelement 52 führbar, um die Messsonde 50 von dem Handstück 1 lösen oder es in das Handstück 1 einzuführen zu können. An dem der Schlitzöffnung 27 gegenüberliegenden Ende des Führungsschlitzes 15 mündet dieser in den Schlitz 11, so dass die beiden Schlitze 11, 15 miteinander verbunden sind. Der, insbesondere kreisbogenförmige, Schlitz 11 erstreckt sich in einem Winkel von etwa 90° zu gleichen Teilen seitlich vom Ende des Führungsschlitzes 15.

Die Außenhülse 2 weist sich in Richtung des Fortsatzes 20 verjüngende Außenflächen auf, die zum Beispiel durch die abgeflachten Bereiche 2A und die Frontseite 16 gebildet sind. Die sich verjüngenden Außenflächen 2A, 16 liegen an einer Fortsatzöffnung 28 an und/ oder begrenzen diese. Der Fortsatz 20 erstreckt sich durch und/ oder aus der Fortsatzöffnung 28 des Handstücks 1. Der Fortsatz 20 erstreckt sich entlang, jedoch antiparallel oder gewinkelt in einem Winkel ungleich 0° relativ zu der Längsachse 21 des Handstücks. Der Fortsatz 20 ist aus Metall, insbesondere Stahl, gefertigt, die Außenhülse 2 aus Kunststoff. Der Fortsatz 20 am ersten Ende 43 des Handstücks 1 weist eine geringere Außenabmessung als die Außenhülse 2 auf, zum Beispiel einen geringeren Außendurchmesser oder einen geringeren Außenumfang, bezogen auf eine im rechten Winkel zu einer Längs- oder Mittelachse des Fortsatzes 20 und der Außenhülse 2 angeordnete Querschnittsfläche durch den Fortsatz 20 und die Außenhülse 2.

In einer Außen- oder Mantelseite des Fortsatzes 20, insbesondere an jener Außen- oder Mantelseite, welche der Messsonde 50, insbesondere dem Messabschnitt 53, der Frontseite 16, dem Sondenraum 19 und/ oder der Öffnung 26 zugewandt ist, befinden sich die Bildaufnahmeöffnung 4 und die Beleuchtungsöffnung 24.

Durch die Bildaufnahmeöffnung 4 gelangt Strahlung von einem Zielobjekt, insbesondere einer Zahnfleischtasche und der Messsonde 50; zu der Bildaufnahmevorrichtung 3 zur Erzeugung der Bildsignale. Durch die Beleuchtungsöffnung 24 gelangt Strahlung, insbesondere sichtbares Licht, von der Beleuchtungsvorrichtung 23 in das Sichtfeld 17 der Bildaufnahmevorrichtung 3 und zu dem Zielobjekt. Die Bildaufnahmeöffnung 4 und die Beleuchtungsöffnung 24 sind getrennte, nebeneinander angeordnete Öffnungen.

Im Inneren des hohlen Fortsatzes 20 ist eine Platine 22 angeordnet ist. Die Platine 22 trägt unter anderem zumindest Teile der Bildaufnahmevorrichtung 3, der Beleuchtungsvorrichtung 23, der Übertragungsvorrichtung 5 und gegebenenfalls weitere elektronische Bauteile des Handstücks 1. Die Platine 22 erstreckt sich aus dem Fortsatz 20 in den Innenraum 35 der Außenhülse 2, vorzugsweise in einem Winkel ungleich 0° relativ zur Längsachse 21 der Außenhülse 2.

Das Innere des Fortsatzes 20 und zumindest ein Teil der Außenhülse 2 ist mit einem Vergussmaterial 25 gefüllt, wobei aus Gründen der Übersichtlichkeit in der Figur 3 nur der Fortsatz 20 gefüllt mit Vergussmaterial 25 dargestellt ist. Die Vergussmasse 25 dient insbesondere auch dazu den Fortsatz unlösbar und dicht mit der Außenhülse zu verbinden.

Figur 6 zeigt eine Messsonde 50 zur Messung parodontaler Messwerte, insbesondere der Tiefe von Zahnfleischtaschen, die mit einem parodontalen Handstück, insbesondere mit den in den Figuren 1 - 5 dargestellten Handstücken 1, verbindbar ist. Wenn die Messsonde 50 in der Lagerausnehmung 7 des Handstücks 1 aufgenommen ist, dann kann sie darin verschwenkt werden, wie dies im Vorstehenden beschrieben ist. Die Messsonde 50 umfasst einen Lagerabschnitt 51, der verschwenkbar in der Lagerausnehmung 7 aufnehmbar ist. An dem Lagerabschnitt 51 ist ein Stellelement 52 vorgesehen, das durch die Öffnung 11 für das Stellelement 52 ragt und darin verschwenkbar geführt ist, um somit die Messsonde 50 auf dem Kreisbogen 9 um die Außenhülse 2 zu verschwenken.

Der Messabschnitt 53 weist eine erste Biegung 54 auf, so dass der Messabschnitt 53 in einem Winkel ungleich 0° relativ zu dem Lagerabschnitt 51 angeordnet ist, um dem Anwender das Messen eines parodontalen Messwertes in der Mundhöhle eines Patienten zu erleichtern. Vorzugsweise ist eine zweite Biegung 56 am Messabschnitt 53 vorgesehen, um insbesondere den Vorderbereich oder die Spitze des Messabschnitts ideal zum Messen eines parodontalen Messwertes zu orientieren.

Der Lagerabschnitt 51 ist länglich zylindrisch geformt. An einem ersten Ende ist er mit dem Messabschnitt 53 verbunden. An dem anderen, gegenüberliegenden Ende des Lagerabschnitts ist eine Anlagefläche 55 vorgesehen ist, an der die Haltevorrichtung 13 des Handstück 1 die Messsonde 50 kontaktiert, um die Messsonde 50 in dem Handstück 1, insbesondere in unterschiedlichen Verschwenkpositionen relativ zu dem Handstück 1, zu halten. Siehe dazu auch die Figuren 4 und 5, in denen die Messsonde 50, fixiert durch die Haltevorrichtung 13 jeweils in zwei unterschiedlichen Verschwenkpositionen entlang des Kreisbogens 9 dargestellt ist. In den rechten Abbildungen der Figuren 4 und 5 ist die Messsonde 50, insbesondere der Messabschnitt 53, dabei jeweils in ihrer maximalen Verschwenkung dargestellt, d.h. das Stellelement 52 ist bis an das jeweilige Ende des kreisbogenförmigen Schlitzes 11 bewegt und/ oder berührt den durch die Außenhülse 2 gebildeten Rand des Schlitzes 11. In den linken Abbildungen der Figuren 4 und 5 ist die Messsonde 50 jeweils nicht maximal verschwenkt, d.h. die Messsonde ist in einer Zwischenposition zwischen der maximalen Verschwenkposition gemäß den rechten Abbildungen und einer Neutral- oder Löseposition, in welcher das Stellelement 52 der Messsonde 50 mit dem Führungsschlitz 15 fluchtet oder darin aufgenommen ist, dargestellt.

An dem Lagerabschnitt 51 ist des Weiteren ein Stellelement 52 vorgesehen, um die Messsonde 50, wie im Vorstehenden beschrieben, relativ zu dem Handstück 1 auf dem Kreisbogen 9 um die Außenhülse 2 des Handstücks 1 zu verschwenken. Das einteilig mit dem Lagerabschnitt 51 ausgebildete Stellelement 52 umfasst einen länglichen Stift mit einem kugeligen Ende. Das Stellelement 52, insbesondere der Stift, erstreckt sich im Wesentlichen rechtwinkelig von dem Lagerabschnitt 51.

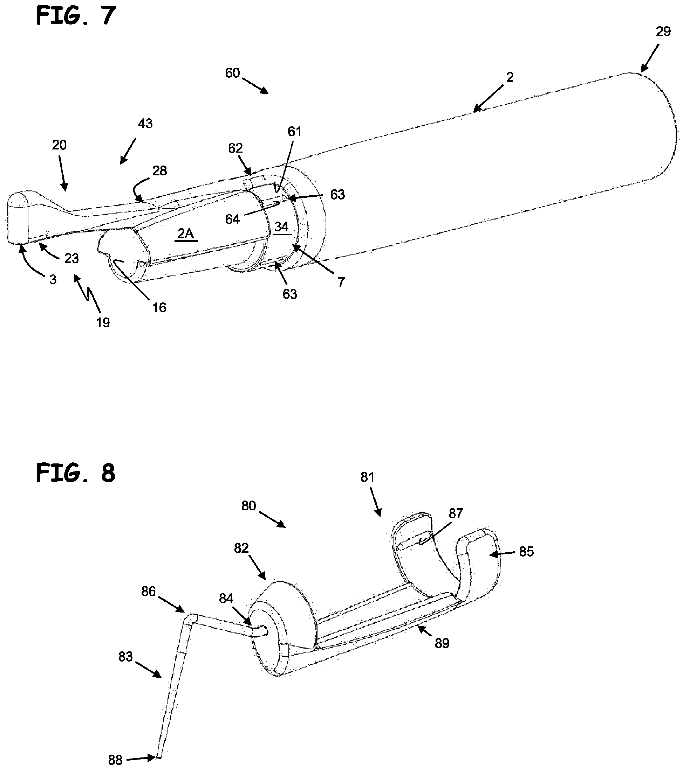

Im Folgenden wird die Lagerausnehmung 7 des Handstücks 60 der Figur 7 beschrieben. Die Lagerausnehmung 7 ist an der Außenseite des Handstücks 60, insbesondere an oder als Teil der Außenhülse 2 vorgesehen. Die Lagerausnehmung 7 umfasst eine kreisbogenförmige Nut 61, in welcher ein Verbindungsabschnitt oder Lagerabschnitt 81 der Messsonde 80 aufnehmbar ist. Die Enden der kreisbogenförmigen Lagerausnehmung 7 sind durch einen Steg 62 voneinander getrennt, der insbesondere von der Außenhülse 2 gebildet wird. Der Steg 62 begrenzt die Verschwenkbarkeit der Messsonde 80 und verhindert damit in vorteilhafter Weise, dass die Messsonde 80, insbesondere der Messabschnitt 83, aus dem Sichtfeld 17 der Bildaufnahmevorrichtung 3 (siehe Figuren 3 - 5) bewegt wird.

An dem Handstück 60, insbesondere an der Lagerausnehmung 7 ist eine Haltevorrichtung 63 vorgesehen, die ausgebildet ist, die Messsonde 80 in unterschiedlichen Verschwenkpositionen entlang des Kreisbogens 9 (entsprechend Figuren 4, 5) zu halten. Damit wird dem Anwender in vorteilhafter Weise das Einführen der Messsonde 80 in eine Zahnfleischtasche erleichtert, da durch die Haltevorrichtung 63 die Messsonde 80 nicht oder schwerer aus ihrer gewählten Verschwenkposition bewegbar ist.

Die Haltevorrichtung 63 ist in der Nut 61 und/ oder an der Zwischenwand 34 angeordnet. Die Haltevorrichtung 63 gemäß Figur 7 ist als mechanische, insbesondere formschlüssige, Haltevorrichtung ausgebildet und umfasst eine Rastverbindung mit in der Zwischenwand 34 angeordneten Rücksprüngen 64, in die ein oder mehrere Vorsprünge 87 der Messsonde 80, insbesondere an dem Verbindungsabschnitt oder Lagerabschnitt 81, eingreifen. Selbstverständlich können die Rücksprünge 64 auch an der Messsonde 80 und die Vorsprünge 87 an der Lagerausnehmung 7 vorgesehen sein.

Wie aus der Figur 7 erkennbar ist, umfasst die Haltevorrichtung 63 mehrere Rücksprünge 64, die um den Umfang der Lagerausnehmung 7, vorzugsweise in regelmäßigen Abständen, angeordnet sind. An der Messsonde 80, insbesondere an der Innenseite des Lagerabschnitts 81, sind mehrere, zum Beispiel zwei Vorsprünge 87 vorgesehen. Gemäß dem Ausführungsbeispiel der Figuren 7 und 8 stehen die zwei Vorsprünge 87 einander in einem Winkel von 180° gegenüber, während die Nut 61 bzw. die Rücksprünge 64 das Handstück 60 in einem Winkel von 270° umgeben, so dass zwischen diesen beiden Winkelmaßen eine Differenz von 90° besteht. Somit kann die Messsonde 80 durch Verschwenken und Einrasten der Vorsprünge 87 in unterschiedlichen Rücksprüngen 64 in unterschiedlichen Verschwenkpositionen entlang des Kreisbogens 9 an dem Handstück 60 befestigt werden, bis zu einer maximalen Verschwenkung von 45° in entgegengesetzten Richtungen aufgrund der Differenz von 90° der beiden Winkelmaße und bezogen auf eine Ausgangsposition, in der eine freie Spitze 88 des Messabschnitts 83 in gleicher Ebene mit oder unterhalb der Bildaufnahmevorrichtung 3 angeordnet ist. Selbstverständlich sind die im Vorstehenden angeführten Winkelmaße exemplarisch und es sind im Rahmen der Erfindung auch andere Winkelmaße denkbar, zum Beispiel derart, dass die maximale Verschwenkung in entgegengesetzte Richtungen unterschiedlich zu 45° ist und zum Beispiel je Richtung 30°, 35°, 40°, 50° oder 55° oder andere Werte beträgt. Es ist auch denkbar, dass die Nut 61 und/ oder die Rücksprünge 64 derart ausgebildet ist/ sind, dass in Bezug auf die Ausgangsposition unterschiedliche Verschwenkwinkel in entgegengesetzte Richtungen möglich sind, zum Beispiel 45° in eine erste Richtung und 55° in die entgegengesetzte Richtung.

Wie aus den Figuren 7 und 8 auch erkennbar ist, ist/ sind die Lagerausnehmung 7 und/ oder die Haltevorrichtung 63 derart ausgebildet, dass die in der Lagerausnehmung 7 aufgenommene Messsonde 80 verschwenkbar ist, während der Lagerabschnitt 81 in der Lagerausnehmung 7 oder Nut 61 aufgenommen ist. Der Anwender muss daher die Messsonde 80 in vorteilhafter Weise nicht aus der Lagerausnehmung 7 entnehmen, um sie zu verschwenken.

Figur 8 zeigt eine Messsonde 80 zur Messung parodontaler Messwerte, insbesondere der Tiefe von Zahnfleischtaschen, die mit einem parodontalen Handstück, insbesondere mit dem in der Figur 7 dargestellten Handstück 1, verbindbar ist. Die Messsonde 80 umfasst einen Lagerabschnitt 81, der verschwenkbar in der Lagerausnehmung 7 aufnehmbar ist. An der Innenseite des Lagerabschnitts 81 ist zumindest ein Rastelement, zum Beispiel Vorsprung 87, vorgesehen, der, wie im Vorstehenden beschrieben, zum Einrasten in die Rücksprünge 64 der Lagerausnehmung 7 ausgebildet ist.

Der Messabschnitt 83 weist eine erste Biegung 84 auf, so dass der Messabschnitt 83 in einem Winkel ungleich 0° relativ zu dem Lagerabschnitt 81 angeordnet ist, um dem Anwender das Messen eines parodontalen Messwertes in der Mundhöhle eines Patienten zu erleichtern. Vorzugsweise ist eine zweite Biegung 86 am Messabschnitt 83 vorgesehen, um insbesondere den Vorderbereich oder die Spitze 88 des Messabschnitts 83 ideal zum Messen eines parodontalen Messwertes zu orientieren.

Der Lagerabschnitt 81 weist an einem Ende eine kappenartige Struktur 82 auf, die, wenn die Messsonde 80 an dem Handstück 60 befestigt ist, die Frontseite 16 des Handstücks 16 umgibt und/ oder kontaktiert und damit vorzugsweise die Messsonde 80 zusätzlich an dem Handstück 60 lagert. Das zumindest eine Rastelement 87 ist auf einem kreisbogenförmigen, vorzugsweise federnd ausgebildeten, Träger 85, insbesondere an dessen Innenseite, angeordnet. Ein, vorzugsweise gebogen ausgebildeter, Steg 89 verbindet die kappenartige Struktur 82 mit dem Träger 85. Der Träger 85 ist auch als Stellelement ausgebildet, das ein Anwender ergreifen kann, um die Messsonde 80 zu verschwenken.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Handstück (1, 60) zur Bestimmung parodontaler Messwerte, insbesondere der Tiefe von Zahnfleischtaschen, umfassend: eine Außenhülse (2), eine Bildaufnahmevorrichtung (3), welche durch eine Bildaufnahmeöffnung (4) des Handstücks (1, 60) Bilder eines Zielobjekts aufnimmt, eine Übertragungsvorrichtung (5) zur Übertragung der durch die Bildaufnahmevorrichtung (3) aufgenommenen Bilder an eine Auswerte- und/ oder Anzeigeeinheit (6) und eine Lagerausnehmung (7) für eine Messsonde (50, 80), **dadurch gekennzeichnet, dass** die Lagerausnehmung (7) derart ausgebildet ist, dass die Messsonde (50, 80) auf einem Kreisbogen (9) um die Außenhülse (2) verschwenkbar ist.

2. Handstück (1, 60) nach Anspruch 1, **dadurch gekennzeichnet, dass** in der Außenhülse (2) eine Öffnung (11) für ein Stellelement (52) vorgesehen ist, um ein Stellelement (52) der Messsonde (50, 80) zum Verschwenken der Messsonde (50, 80) auf dem Kreisbogen (9) aufzunehmen und/ oder zu führen.

3. Handstück (1, 60) nach einem der vorstehenden Ansprüche, **dadurch gekennzeichnet, dass**
das Handstück (1, 60) eine Haltevorrichtung (13, 63) aufweist, um die Messsonde (50, 80) in der Lagerausnehmung (7) in unterschiedlichen Verschwenkpositionen entlang des Kreisbogens (9) zu halten.

4. Handstück (1, 60) nach Anspruch 3, **dadurch gekennzeichnet, dass** die Haltevorrichtung (13, 63) in einer an die Lagerausnehmung (7) anschließenden Aufnahme (14) angeordnet ist.

5. Handstück (1, 60) nach Anspruch 3 oder 4, **dadurch gekennzeichnet, dass** die Haltevorrichtung (13, 63) derart ausgebildet ist, dass die in der Lagerausnehmung (7) aufgenommene Messsonde (50, 80) verschwenkbar ist, die Messsonde (50, 80) in der Lagerausnehmung (7) aufgenommen ist und/ oder während die Haltevorrichtung (13) die Messsonde (50, 80) in der Lagerausnehmung (7) hält.

6. Handstück (1, 60) nach einem der vorstehenden Ansprüche 2 - 5, **dadurch gekennzeichnet, dass**
die Öffnung (11) für das Stellelement (52) als Widerlager (44) ausgebildet ist, welches durch die Haltevorrichtung (13, 63) auf die Messsonde (50, 80) ausgeübte Kräfte aufnimmt.

7. Handstück (1, 60) nach Ansprüche 2 - 6, **dadurch gekennzeichnet, dass** ein Führungsschlitz (15) in der Außenhülse (2) sich von der Öffnung (11) für das Stellelement (52) der Messsonde (50, 80) bis zu einer Frontseite (16) des Handstücks (1, 60) erstreckt, durch welchen das Stellelement (52) der Messsonde (50, 80) führbar ist, um die Messsonde (50, 80) von dem Handstück (1, 60) zu lösen.

8. Handstück (1, 60) nach einem der vorstehenden Ansprüche, **dadurch gekennzeichnet, dass**
die Lagerausnehmung (7) und/ oder die Öffnung (11) für das Stellelement (52) der Messsonde (50, 80) derart bemessen ist, dass durch das Betätigen des Stellelements (52) der Messabschnitt (53) der Messsonde (50, 80) nur innerhalb des Sichtfelds (17) der Bildaufnahmevorrichtung (3) verschwenkbar ist.

9. Handstück (1, 60) nach einem der vorstehenden Ansprüche, **dadurch gekennzeichnet, dass**
die Bildaufnahmeöffnung (4) in einem Fortsatz (20) an einem Ende der Außenhülse (2) angeordnet ist, wobei sich die Messsonde (50, 80), insbesondere ein Messabschnitt (53, 83) der Messsonde (50, 80), an dem Fortsatz (20) erstreckt.

10. Handstück (1, 60) nach Anspruch 9, **dadurch gekennzeichnet, dass** der Fortsatz (20) an einem ersten Ende (43) der Außenhülse (2) angeordnet ist, das sich in Richtung des Fortsatzes (20) verjüngende Außenflächen (2A, 16) der Außenhülse (2) umfasst.

11. Handstück (1, 60) nach Anspruch 9 oder 10, **dadurch gekennzeichnet, dass** der Fortsatz (20) und eine Frontseite (16) der Außenhülse (2) einen außerhalb des Handstücks (1, 60) angeordneten Sondenraum (19) definieren, der von dem Fortsatz (20) überragt ist und in dem zumindest ein Teil der Messsonde (50, 80) angeordnet ist.

12. Handstück (1, 60) nach einem der Ansprüche 9 - 11, **dadurch gekennzeichnet, dass** sich der Fortsatz (20) in einem Winkel ungleich 0° relativ zu einer Längsachse (21) der Außenhülse (2) erstreckt.

13. Handstück (1, 60) nach Anspruch 12, **dadurch gekennzeichnet, dass** in dem Fortsatz (20) eine Platine (22) angeordnet ist, die sich in die Außenhülse (2) in einem Winkel ungleich 0° relativ zu einer Längsachse (21) der Außenhülse (2) erstreckt.

14. Handstück (1, 60) nach einem der vorstehenden Ansprüche, **dadurch gekennzeichnet, dass**
das Handstück (1, 60) eine Messsonde (50, 80) aufweist, die einen Lagerabschnitt (51, 81) aufweist, der verschwenkbar in der Lagerausnehmung (7) des Handstücks (1, 60) angeordnet ist, wobei bevorzugt die Messsonde (50, 80), insbesondere der Lagerabschnitt (51), ein Stellelement (52) aufweist, das durch die Öffnung (11) für das Stellelement (52) ragt und relativ zur Außenhülse (2) verschwenkbar ist, um die Messsonde (50, 80) auf dem Kreisbogen (9) um die Außenhülse (2) zu verschwenken.

15. Handstück (1, 60) nach Anspruch 14, **dadurch gekennzeichnet, dass** sich die Messsonde (50, 80), insbesondere ein Messabschnitt (53, 83) der Messsonde (50, 80), und/ oder das Sichtfeld (17) der Bildaufnahmevorrichtung (3) in einem Winkel ungleich 0° von einer Längsachse (21) der Außenhülse (2) erstrecken.

16. Handstück (1, 60) zur Bestimmung parodontaler Messwerte, insbesondere der Tiefe von Zahnfleischtaschen, umfassend: eine Außenhülse (2), eine Bildaufnahmevorrichtung (3), welche durch eine Bildaufnahmeöffnung (4) des Handstücks (1, 60) Bilder eines Zielobjekts aufnimmt, eine Übertragungsvorrichtung (5) zur Übertragung der durch die Bildaufnahmevorrichtung (3) aufgenommenen Bilder an eine Auswerte- und/ oder Anzeigeeinheit (6) und eine Lagerausnehmung (7) für eine Messsonde (50, 80) oder Handstück (1, 60) nach einem der vorstehenden Ansprüche 1 - 15, **dadurch gekennzeichnet, dass**
die Bildaufnahmeöffnung (4) in einem Fortsatz (20) an einem Ende der Außenhülse (2) angeordnet ist, wobei das Ende der Außenhülse (2) sich in Richtung des Fortsatzes (20) verjüngende Außenflächen (2A, 16) der Außenhülse (2) umfasst.

17. Handstück (1, 60) nach Anspruch 16, **dadurch gekennzeichnet, dass** der Fortsatz (20) und eine der sich in Richtung des Fortsatzes (20) verjüngenden Außenflächen (2A, 16) einen Sondenraum (19) definieren, der von dem Fortsatz (20) überragt ist und in dem zumindest ein Teil der Messsonde (50, 80) anordenbar ist.

18. Handstück (1, 60) nach Anspruch 17, **dadurch gekennzeichnet, dass** zumindest ein Teil der Messsonde (50, 80) in dem Sondenraum (19), auf einem Kreisbogen (9) um die Außenhülse (2) verschwenkbar an dem Handstück (1, 60) angeordnet ist.

19. Handstück (1, 60) nach einem der Ansprüche 16 - 18, **dadurch gekennzeichnet, dass** sich der Fortsatz (20) in einem Winkel ungleich 0° relativ zu einer Längsachse (21) der Außenhülse (2) erstreckt.

20. Handstück (1, 60) nach einem der Ansprüche 16 - 19, **dadurch gekennzeichnet, dass** in dem Fortsatz (20) zumindest ein Teil der Bildaufnahmevorrichtung (3) angeordnet ist.

21. Handstück (1, 60) nach einem der Ansprüche 16 - 20, **dadurch gekennzeichnet, dass** in dem Fortsatz (20) zumindest ein Teil einer Beleuchtungsvorrichtung (23) zur Abgabe elektromagnetischer Strahlung zur Beleuchtung des Zielobjekts vorgesehen ist.

22. Handstück (1, 60) nach Anspruch 21, **dadurch gekennzeichnet, dass** in dem Fortsatz (20) eine Beleuchtungsöffnung (24) zur Abgabe elektromagnetischer Strahlung durch die Beleuchtungsvorrichtung (23) in den Sondenraum (19) und/ oder in Richtung der Messsonde (50, 80) vorgesehen ist.

23. Handstück (1, 60) nach einem der Ansprüche 16 - 22, **dadurch gekennzeichnet, dass** in dem Fortsatz (20) eine Platine (22) angeordnet ist, die sich in dem Fortsatz (20) und in der Außenhülse (2) in einem Winkel ungleich 0° relativ zu einer Längsachse (21) der Außenhülse (2) erstreckt.

24. Handstück (1, 60) nach Anspruch 23, **dadurch gekennzeichnet, dass** zumindest ein Teil der Bildaufnahmevorrichtung (3) und/ oder der Beleuchtungsvorrichtung (23) auf der Platine (22) angeordnet ist/sind.

25. Handstück (1, 60) nach einem der Ansprüche 16 - 25, **dadurch gekennzeichnet, dass** der Fortsatz (20) eine metallische Außenhülse (2) umfasst und/ oder dass die Außenhülse (2) des Handstücks (1, 60) aus Kunststoff gefertigt ist.

26. Handstück (1, 60) nach einem der Ansprüche 16 - 25, **dadurch gekennzeichnet, dass** das Innere des Fortsatzes (20) und/ oder zumindest ein Teil der Außenhülse (2) mit einem Vergussmaterial (25) gefüllt ist.

27. Messsonde (50, 80) zur Bestimmung parodontaler Messwerte, insbesondere der Tiefe von Zahnfleischtaschen, oder Handstück (1, 60) nach einem der vorstehenden Ansprüche, **dadurch gekennzeichnet, dass**
die Messsonde (50, 80) einen Lagerabschnitt (51, 81) zur Lagerung der Messsonde (50, 80) an dem Handstück (1, 60) und einen daran anschließenden Messabschnitt (53, 83) zur Bestimmung parodontaler Messwerte, insbesondere der Tiefe von Zahnfleischtaschen, umfasst, wobei der Messabschnitt (53, 83) zumindest eine Biegung (54, 56; 84, 86) aufweist, so dass der Messabschnitt (53, 83) in einem Winkel ungleich 0° relativ zu dem Lagerabschnitt (51, 81) angeordnet ist.

28. Messsonde (50, 80) oder Handstück (1, 60) nach Anspruch 27, **dadurch gekennzeichnet, dass**
die Messsonde (50, 80), insbesondere der Lagerabschnitt (51, 81), ein Stellelement (52, 85) aufweist, um die Messsonde (50, 80) relativ zu dem Handstück (1, 60), insbesondere auf dem Kreisbogen (9) um die Außenhülse (2) des Handstücks (1, 60), zu verschwenken.

29. Messsonde (50, 80) oder Handstück (1, 60) nach Anspruch 28, **dadurch gekennzeichnet, dass**
das Stellelement (52) sich im Wesentlichen rechtwinkelig von dem Lagerabschnitt (51) erstreckt.

30. Messsonde (50, 80) oder Handstück (1, 60) nach einem der Ansprüche 27 - 29, **dadurch gekennzeichnet, dass**
an dem Ende des Lagerabschnitts (51), das dem Messabschnitt (53) gegenüber angeordnet ist, eine Anlagefläche (55) vorgesehen ist, an der eine Haltevorrichtung (13) des Handstück (1, 60) die Messsonde (50, 80) kontaktiert, um die Messsonde (50, 80) in dem Handstück (1, 60), insbesondere in unterschiedlichen Verschwenkpositionen relativ zu dem Handstück (1, 60), zu halten.
